# EUROPEAN PATENT APPLICATION

(11) **EP 1 361 213 A1**
(43) Date of publication of application: **12.11.2003**
(21) Application number: 02734832.5
(22) Date of filing: 22.01.2002
(51) Int. Cl.: C07D 211/26, A61K 31/445

(54) **PROCESS FOR PREPARATION OF CYANOPHENYLBENZOIC ACID DERIVATIVES**

(30) Priority: 26.01.2001 JP 2001018224
(71) Applicant: TEIJIN LIMITED, Osaka-shi Osaka 541-0054 (JP)
(72) Inventor: HARA, Takayuki, Teijin Limited, Tokyo Research Ctr, Hino-shi, Tokyo 191-0065 (JP); MINOSHIMA, Toru, Teijin Limited, Iwakuni Factory, Iwakuni-shi, Yamaguchi 740-0014 (JP); TABE, Masayasu, Teijin Limited, Chiyoda-ku, Tokyo 100-0011 (JP)
(74) Representative: Hallybone, Huw George
(86) International application number: JP0200437
(87) International publication number: WO02059091

(57) **Abstract**

A process for the production of a compound represented by the following Formula (IV) or a salt thereof, comprising the steps of condensing with dehydration a compound represent by the following Formula (I) and 4-(aminomethyl)piperidine to obtain a compound represented by the following Formula (II); protecting the secondary amine of the piperidine moiety to give a compound represented by the following Formula (III); and then reducing the imine moiety (in the Formulas, the wavy line may be, relative to the double bond, any of an E form, a Z form, or a mixture thereof, R represents a hydrogen atom, a C1-8 alkyl group etc., and R' represents a C1-8 alkylcarbonyl group, an arylcarbonyl group etc.).

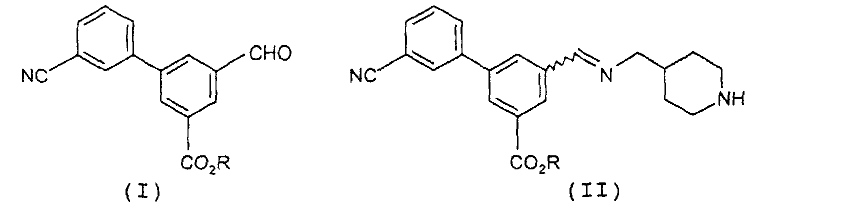

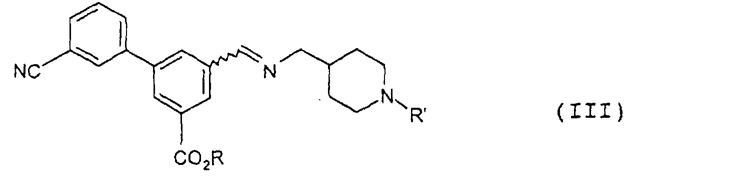

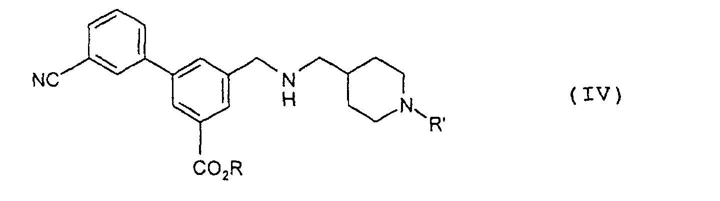

## Description

### Field of the Invention

The present invention relates to a process for production of 3-(3-cyanophenyl)-5-({[(4-piperidyl)methyl]amino}methyl)benzoic acid derivatives represented by Formula (IV): 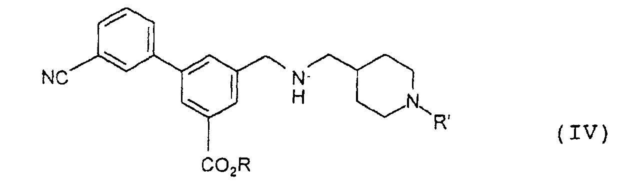 and the salts thereof. More specifically, it relates to a method of preparing 3-(3-cyanophenyl)-5-({[(4-piperidyl)methyl)amino}methyl)benzoic acid derivatives or salts thereof, useful for use as intermediates for the production of a novel selective inhibitor of activated blood coagulation factor X (hereinafter referred to as FXa).

### Background Art

As agents for inhibiting blood clots, anti-thrombin agents have, conventionally, been developed. However, as the anti-thrombin agents have been known to inhibit the agglutination of platelets by thrombin as well as to inhibit blood coagulation, and thereby to have a risk of inducing a bleeding tendency, they could not be easily used to control coagulation. Thus, attempts have been made to develop anti-coagulation agents whose action mechanism is based on effects other than the effect of inhibiting thrombin, and these attempts led to the discovery of biphenylamidine derivatives, described in International Patent Publication WO 99/26918, as an anticoagulant having an excellent effect of inhibiting FXa.

Among them, intermediates 3-(3-cyanophenyl)-5-({[(1-tert-butoxycarbonyl-4-piperidyl)methyl]amino}methyl)benzoic acid derivatives can be obtained by bromating the hydroxymethyl group of 3-(3-cyanophenyl)-5-(hydroxymethyl)benzoic acid derivatives with phosphorus tribromide in diethylether thereby to convert it to the corresponding bromomethyl group, and the products are then added to 4-aminomethyl-1-(tert-butoxycarbonyl)piperidine (see the specification of WO 99/26918).

4-aminomethyl-1-(tert-butoxycarbonyl)piperidine used here must be prepared beforehand in a process wherein benzaldehyde is first allowed to react to 4-aminomethyl piperidine to protect the primary amino group by imination, then the secondary amino group of the piperidine ring is tert-butoxycarbonylated, and finally it is deiminated (deprotected).

On the other hand, International Patent Publication WO 00/69811 describes a method of preparing 3-(3-cyanophenyl)-5-({[(1-tert-butoxycarbonyl-4-piperidyl)methyl]amino}methyl)benzoic acid derivatives by imine formation using 4-aminomethyl-1-(tert-butoxycarbonyl)piperidine and the subsequent reduction reaction. In this case also, as in International Patent Publication WO 99/26918, 4-aminomethyl-1-(tert-butoxycarbonyl)piperidine must be prepared beforehand. Furthermore, due to many reaction steps in both of the preparation methods, the overall yield of the desired intermediate 3-(3-cyanophenyl)-5-({[(1-tert-butoxycarbonyl-4-piperidyl)methyl]amino}methyl)benzoic acid derivatives remained at moderate levels.

From the foregoing, the above-mentioned conventional methods have the following drawbacks: 1) a special flammable agent, diethylether, whose handling is dangerous is used at bromation, and 2) multiple steps such as amino group protection and deprotection are required in the preparation of raw material compounds.

Thus, these methods are far from satisfactory as methods for industrial production, and thus there has been a need for the development of production methods that replace the above conventional methods requiring dangerous solvents and multi-step preparation of raw material compounds.

### Disclosure of the Invention

Considering the above-mentioned conventional methods, it is an object of the present invention to provide a method of preparing 3-(3-cyanophenyl)-5-({[(4-piperidyl )methyl ] amino}methyl )benzoic acid derivatives in simple and efficient processes without using dangerous solvents such as special flammables.

After intensive and extensive efforts to attain the above objective, the present inventors have discovered an efficient method of preparation, shown below, wherein the reductive amination and the protection of amino group of the piperidine ring are carried out simultaneously in a series of processes, and thereby have completed the present invention.

Thus, the present invention provides a process for production of a 3-(3-cyanophenyl)-5-({[(4-piperidyl)methyl]amino}methyl)benzoic acid derivative renresented by the following Formula (IV): 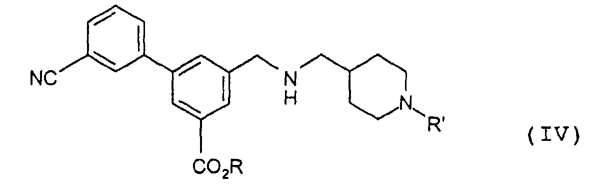 [wherein
R represents a hydrogen atom, a C1-8 alkyl group, an aryl group, or an aralkyl group, and
R' represents a C1-8 alkylcarbonyl group, an arylcarbonyl group, a C1-8 alkoxycarbonyl group, an aryloxycarbonyl group, or an aralkoxycarbonyl group]
or a salt thereof,
comprising the steps of:
condensing with dehydration a 3-(3-cyanophenyl)-5-formyl benzoic acid derivative represented by the following Formula (I): 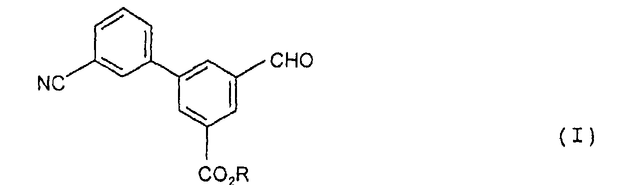
[wherein
R represents a hydrogen atom, a C1-8 alkyl group, an aryl group, or an aralkyl group]
and 4-(aminomethyl)piperidine to obtain a 3-(3-cyanophenyl)-5-({N-[(4-piperidyl)methyl]imino}methyl )benzoic acid derivative represented by the following Formula (II): 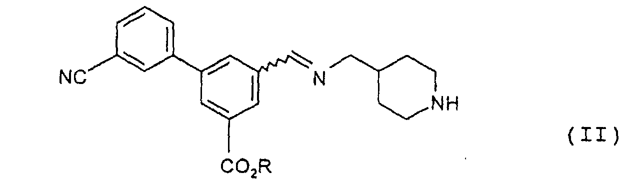 [wherein the wavy line may be, relative to the double bond, any of an E form, a Z form, or a mixture thereof at any ratio, and
R represents a hydrogen atom, a C1-8 alkyl group, an aryl group, or an aralkyl group];
protecting the secondary amine of the piperidine moiety to give a 3-(3-cyanophenyl)-5-({N-[(4-piperidyl)methyl]imino}methyl)benzoic acid derivative, having a protecting group, represented by the following Formula (III): 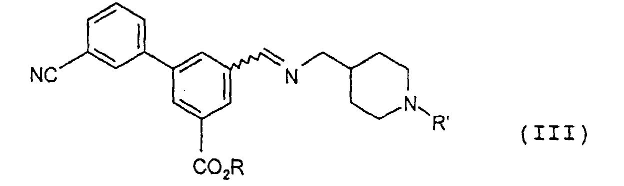 [wherein the wavy line may be, relative to the double bond, any of an E form, a Z form, or a mixture thereof at any ratio,
R represents a hydrogen atom, a C1-8 alkyl group, an aryl group, or an aralkyl group, and
R' represents a C1-8 alkylcarbonyl group, an arylcarbonyl group, a C1-8 alkoxycarbonyl group, an aryloxycarbonyl group, or an aralkoxycarbonyl group]; and then
reducing the imine moiety.

The present invention also provides a process for production of a 3-(3-cyanophenyl)-5-({[(4-piperidyl)methyl]amino}methyl)benzoic acid derivative represented by the following Formula (VIII): 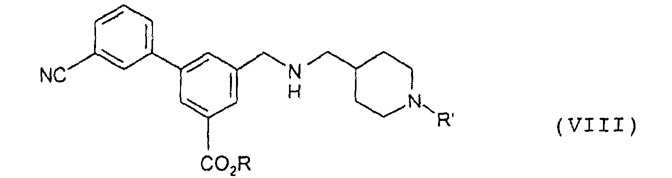 [wherein
R represents a C1-8 alkyl group, and
R' represents a C1-8 alkoxycarbonyl group]
or a salt thereof,
comprising the steps of:
condensing with dehydration a 3-(3-cyanophenyl)-5-formyl benzoic acid derivative represented by the following Formula (V): 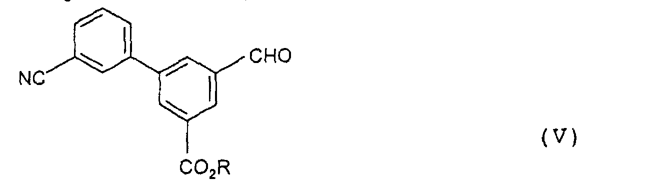
[wherein R represents a C1-8 alkyl group]
and 4-(aminomethyl)piperidine in a toluene solution to give a 3-(3-cyanophenyl)-5-[{N-[(4-piperidyl]methyl]imino}methyl)benzoic acid derivative represented by the following Formula (VI): 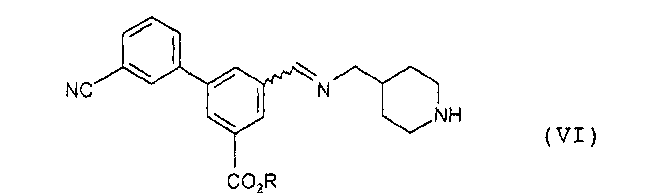 [wherein,
the wavy line may be, relative to the double bond, any of an E form, a Z form, or a mixture thereof at any ratio, and
R represents a C1-8 alkyl group];
protecting the secondary amine of the piperidine moiety to give a 3-(3-cyanophenyl)-5-({N-[(4-piperidyl)methyl]imino}methyl)benzoic acid derivative, having a protecting group, represented by the following Formula (VII): 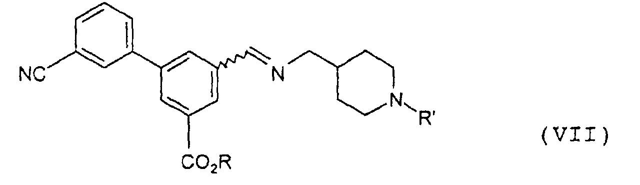 [wherein,
the wavy line may be, relative to the double bond, any of an E form, a Z form, or a mixture thereof at any ratio,
R represents a C1-8 alkyl group, and
R' represents a C1-8 alkoxycarbonyl group]; and then reducing the imine moiety.

The present invention also provides a process for production of a 3-(3-cyanophenyl)-5-({[(1-tert-butoxycarbonyl-4-piperidyl)methyl]amino}methyl)benzoic acid derivative represented by the following Formula (X): 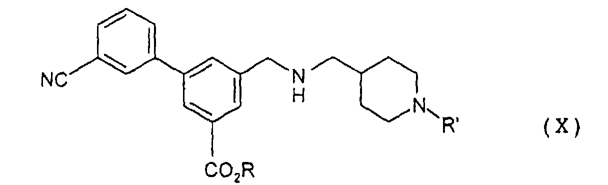 [wherein
R represents a C1-8 alkyl group, and
R' represents a tert-butoxycarbonyl group]
or a salt thereof,
comprising the steps of:
condensing with dehydration a 3-(3-cyanophenyl)-5-formyl benzoic acid derivative represented by the following Formula (V): 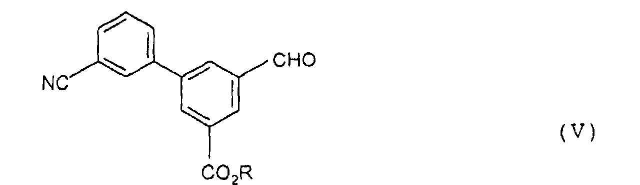
[wherein R represents a C1-8 alkyl group]
and 4-(aminomethyl)piperidine in a toluene solution to give a 3-(3-cyanophenyl)-5-({N-[(4-piperidyl)methyl]imino}methyl)benzoic acid derivative represented by the following Formula (VI): 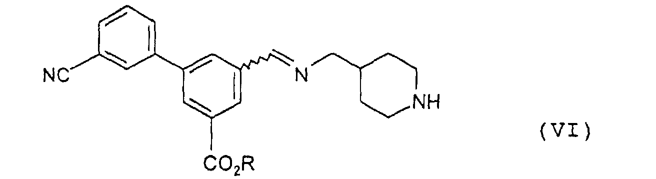 [wherein,
the wavy line may be, relative to the double bond, any of an E form, a Z form, or a mixture thereof at any ratio, and
R represents a C1-8 alkyl group];
protecting the secondary amine of the piperidine moiety with a tert-butoxycarbonyl group to give a 3-(3-cyanophenyl)-5-({N-[(1-tert-butoxycarbonyl-4-piperidyl)methyl]imino}methyl)benzoic acid derivative represented by the following Formula (IX): 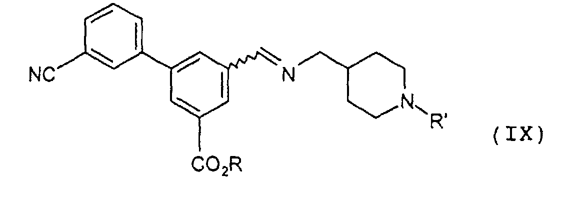 [wherein,
the wavy line may be, relative to the double bond, any of an E form, a Z form, or a mixture thereof at any ratio,
R represents a C1-8 alkyl group, and
R' represents a tert-butoxycarbonyl group]; and then
reducing the imine moiety with a borohydride derivative.

The present invention also provides a novel and useful intermediate 3-(3-cyanophenyl)-5-({N-[(4-piperidyl)methyl]imino}methyl)benzoic acid derivative represented by the following Formula (II): 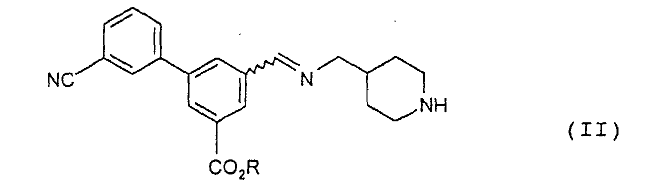 [wherein,
the wavy line may be, relative to the double bond, any of an E form, a Z form, or a mixture thereof at any ratio, and
R represents a hydrogen atom, a C1-8 alkyl group, an aryl group, or an aralkyl group]
and the salt thereof which are obtained by the above preparation method.

### Best Mode for Carrying Out the Invention

In the above formulas, the conversion from (I) to (II) and from (V) to (VI) is carried out removing the water formed by the reaction from the system. Solvents used include, for example, aromatic hydrocarbons such as benzene, toluene and xylene, with toluene being most preferred. Reaction is preferably carried out at, but is not limited to, a relatively high temperature in order to remove the water from the system, and is preferably 80°C to 150°C. The reaction time may vary depending on the solvents and the reaction temperature, and are generally 1 to 24 hours, and preferably 1 to 6 hours.

In the protection reaction of secondary amino group of the piperidine ring from (II) to (III), from (VI) to (VII), and from (VI) to (IX), multi-stage processes such as concentration and dilution may be avoided by adding a protective reagent to the above-mentioned reaction mixture. At this time, the protective reagent may be added after diluting in a suitable solvent that does not affect the reaction. The protective groups are not specifically limited as long as they are generally used for the protection of the amino group and they are stable under the condition of the reduction in the later steps, and include, for example, C1-8 alkylcarbonyl groups, arylcarbonyl groups, C1-8 alkoxycarbonyl groups, aryloxycarbonyl groups, or aralkoxycarbonyl groups, and preferably C1-8 alkoxycarbonyl groups with the tert-butoxycarbonyl group being most preferred. Reaction is preferably carried out at relatively low temperatures in order to control side reactions, and generally at 0 to 30°C.

The reduction from (III) to (IV), from (VII) to (VIII), and from (IX) to (X) may be any method as long as it does not produce byproducts, and there can be used, for example, hydrogenation, metal hydrides, electrolysis and the like. Among them, as metal hydrides for use in reduction, there can be mentioned, for example, borohydride derivatives, and preferably borane, sodium borohydride, and sodium cyanoborohydride are used, with sodium borohydride being most preferred. At this time, in order to avoid cumbersome multi-step processes such as concentration and dilution as described above, the reaction solution may be used without concentration, and activators may be added, as desired, to promote the reaction. The reaction mixture containing (IV) thus obtained may be subjected to simple post-treatment such as extraction to obtain a highly purified (IV) without troublesome purification procedures.

Furthermore, the present invention provides a novel and useful intermediate 3-(3-cyanophenyl)-5-({N-[(4-piperidyl)methyl]imino}methyl)benzoic acid derivative represented by the following Formula (III): 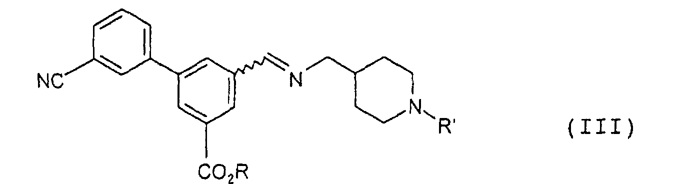 [wherein
the wavy line may be, relative to the double bond, any of an E form, a Z form, or a mixture thereof at any ratio,
R represents a hydrogen atom, a C1-8 alkyl group, an aryl group, or an aralkyl group, and
R' represents a C1-8 alkylcarbonyl group, an arylcarbonyl group, a C1-8 alkoxycarbonyl group, an aryloxycarbonyl group, or an aralkoxycarbonyl group]
and the salts thereof, obtained by the above-mentioned method of preparation.

In the definitions with regard to substituents of the compounds represented by the formulas (I) to (X) of the present invention, "C1-8 alkyl group" means a linear or branched carbon chain having one to eight carbons, and specifically includes a methyl group, an ethyl group, a propyl group, an isopropyl group, a butyl group, an isobutyl group, a sec-butyl group, a tert-butyl group, a pentyl group, a neopentyl group, an isopentyl group, a 1,2-dimethylpropyl group, a hexyl group, an isohexyl group, a 1,1-dimethylbutyl group, a 2,2-dimethylbutyl group, a 1-ethylbutyl group, a 2-ethylbutyl group, an isoheptyl group, an octyl group, or an isooctyl group, and preferably those having one to four carbons, with a methyl group and an ethyl group being most preferred.

"Aryl group" specifically means cyclic hydrocarbon aryl groups such as a phenyl group and a naphthyl group, and heteroaryl groups such as a pyridyl group and a furyl group, with a phenyl group being preferred.

"Aralkyl group" specifically means a benzyl group, a phenethyl group, a phenylpropyl group, a 1-naphthylmethyl group, a 2-naphthylmethyl group etc., with a benzyl group being preferred.

"C1-8 alkylcarbonyl group" means a linear or branched carbon chain having one to eight carbons, and specifically includes a methyl group, an ethyl group, a propyl group, an isopropyl group, a butyl group, an isobutyl group, a sec-butyl group, a tert-butyl group, a pentyl group, a neopentyl group, an isopentyl group, a 1,2-dimethylpropyl group, a hexyl group, an isohexyl group, a 1,1-dimethylbutyl group, a 2,2-dimethylbutyl group, a 1-ethylbutyl group, a 2-ethylbutyl group, an isoheptyl group, an octyl group, or an isooctyl group, and preferably those having one to four carbons, with a methyl group and an ethyl group being most preferred.

As the aryl group in "arylcarbonyl group", there can be mentioned specifically cyclic hydrocarbon aryl groups such as a phenyl group and a naphthyl group, and heteroaryl groups such as a pyridyl group and a furyl group, with a phenyl group being preferred.

As the alkoxy group in "C1-8 alkoxycarbonyl group", there can be mentioned specifically a methoxy group, an ethoxy group, a propoxy group, an isopropoxy group, a butoxy group, an isobutoxy group, a sec-butoxy group, a tert-butoxy group, a pentyloxy group, a neopentyloxy group, an isopentyloxy group, a 1,2-dimethylpropyloxy group, a hexyloxy group, an isohexyloxy group, a 1,1-dimethylbutyloxy group, a 2,2-dimethylbutyloxy group, a 1-ethylbutyloxy group, a 2-ethylbutyloxy group, an isoheptyloxy group, an octyloxy group, an isooctyloxy group, or the like, and preferably those having one to four carbons, with a methoxy group, a ethoxy group and a tert-butoxy group being most preferred.

As the aryl group in "aryloxycarbonyl group", there can be mentioned specifically cyclic hydrocarbon aryl groups such as a phenyl group and a naphthyl group, and heteroaryl groups such as a pyridyl group and a furyl group, with a phenyl group being preferred.

As the aralkoxy group in "aralkoxycarbonyl group", there can be mentioned specifically a benzyloxy group, a phenethyloxy group, a phenylpropyloxy group, a 1-naphthylmethyloxy group, a 2-naphthylmethyloxy group etc., with a benzyloxy group being most preferred.

The wavy line as used herein may be, relative to the double bond with a nitrogen atom, any of an E form, a Z form, or a mixture thereof.

### Examples

The present invention will now be explained more specifically with reference to specific examples. However, it is to be noted that the scope of the present invention is not limited by these examples in any way.

### Example 1. Synthesis of methyl 3-(3-cyanophenyl)-5-({N-[(4-piperidyl)methyl]imino}methyl)benzoate

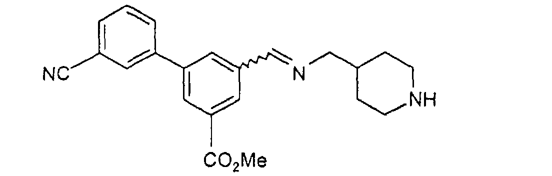

44.5 g of methyl 3-(3-cyanophenyl)-5-formyl benzoate (obtained by the method described in International Patent Publication WO 99/26918) and 19.2 g of 4-(aminomethyl)piperidine were dissolved in 265 ml of toluene, and, while removing water formed as a byproduct by using the Dean-Stark dehydrator, the reaction mixture was heated to reflux at 130°C for 3 hours to give methyl 3-(3-cyanophenyl)-5-({N-[(4-piperidyl)methyl]imino}methyl)benzoate (the above formula (V)). A portion of the reaction mixture was concentrated to dryness, and the structure of the title compound was confirmed by NMR.

1H-NMR (200 MHz, δ ppm, CDCl3) 1.1-1.4 (m, 2H), 1.6-2.0 (m, 3H), 2.64 (dt, J=2.5 & 12.1 Hz, 2H), 3.10 (br.d, J=11.8 Hz, 2H), 3.56 (d, J=6.3 Hz, 2H), 3.98 (S, 3H), 7.5-7.7 (m, 2H), 7.90 (d, J=7.6 Hz, 1H), 7.95 (s, 1H), 8.20 (s, 1H), 8.29 (s, 1H), 8.35 (br.s, 2H).

### Example 2. Synthesis of methyl 3-(3-cyanophenyl)-5-{{N-[(1-tert-butoxycarbonyl-4-piperidyl)methyl]imino}-methyl)benzoate

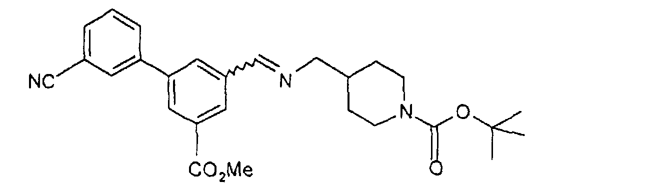

The solution of methyl 3-(3-cyanophenyl)-5-({N-[(4-piperidyl)methyl]imino}methyl)benzoate obtained in Example 1 in toluene was cooled on ice, to which solution 38.5 g of ditert-butyl dicarbonate dissolved in 35 ml of toluene was added dropwise. After dropwise addition, the reaction mixture was stirred at room temperature for 2 hours to give methyl 3-(3-cyanophenyl)-5-({N-[(1-tert-butoxycarbonyl-4-piperidyl)methyl]imino}methyl)benzoate. A portion of the reaction mixture was concentrated to dryness, and the structure of the title compound was confirmed by NMR.

1H-NMR (200 MHz, δ ppm, CDCl3) 1.1-1.4 (m, 2H), 1.46 (s, 9H), 1.6-2.0 (m, 3H), 2.73 (br.t, 2H), 3.56 (d, J=6.5 Hz, 2H), 3.99 (s, 3H), 4.1-4.2 (br.d, 2H), 7.5-7.7 (m, 2H), 7.90 (dd, J=1.6 & 7.8 Hz, 1H), 7.95 (s, 1H), 8.19 (S, 1H), 8.30 (s, 1H), 8.35 (d, J=1.5 Hz, 2H).

### Example 3. Synthesis of methyl 3-(3-cyanophenyl)-5-({[(1-tert-butoxycarbonyl-4-piperidyl)methyl]amino}methyl)benzoate

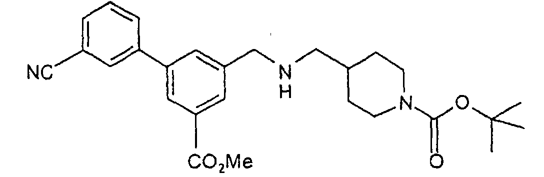

To the solution containing methyl 3-(3-cyanophenyl)-5-({N-[(1-tert-butoxycarbonyl-4-piperidyl)methyl]imino}methyl)benzoate obtained in Example 2, 300 ml of methanol was added, which was then cooled on ice. To the solution was added 6.7 g of sodium borohydride in portions. After the completion of the addition, the reaction mixture was stirred at room temperature for 3 hours. Toluene and water were added to the reaction mixture, and vigorously stirred. After the phases separated, the aqueous layer was extracted with toluene, and washings were combined with the organic layer. The organic solution obtained was washed with water and brine, and then dried over anhydrous sodium sulfate. The solution was concentrated under reduced pressure to give 70.0 g of methyl 3-(3-cyanophenyl)-5-({[(1-tert-butoxycarbonyl-4 piperidyl)methyl]amino}methyl)benzoate. Using NMR and mass spectrometric analysis, the structure of the title compound was confirmed. The yield from Working Example 1 is 90%.

1H-NMR (200 MHz, δ ppm, CDCl3) 1.0-1.4 (m, 2H), 1.45 (s, 9H), 1.6-1.8 (m, 3H), 2.55 (d, J=6.23 Hz, 2H), 2.70 (brt, J=12.2 Hz, 2H), 3.91 (s, 2H), 3.96 (s, 3H), 4.0-4.2 (m, 2H), 7.5-7.7 (m, 2H), 7.87 (dd, J=1.3 & 6.4 Hz, 1H), 7.90 (s, 1H), 8.04 (s, 1H), 8.13 (s, 1H). [M+H]=464.3.

### Industrial Applicability

According to the present invention, 3-(3-cyanophenyl)-5-({[(4-piperidyl)methyl]amino}methyl)benzoic acid derivatives which are useful intermediates for use in the production of biphenylamidine derivatives, anticoagulants having an excellent FXa-inhibiting effect described in International Patent Publication WO 99/26918, can be produced in a simple and efficient manner without using dangerous flammable organic solvents. Furthermore, in the above method, novel and useful intermediates 3-(3-cyanophenyl)-5-({N-[(4-piperidyl)methyl)imino}methyl)benzoic acid derivatives can be provided.

## Claims

1. A process for production of a 3-(3-cyanophenyl)-5-({[ (4-piperidyl)methyl]amino}methyl)benzoic acid derivative represented by the following Formula (IV): 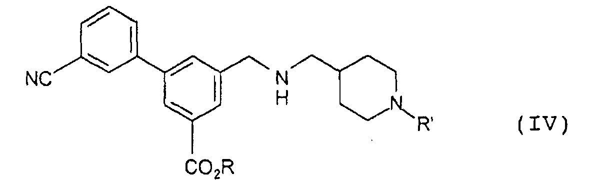 [wherein
R represents a hydrogen atom, a C1-8 alkyl group, an aryl group, or an aralkyl group, and
R' represents a C1-8 alkylcarbonyl group, an arylcarbonyl group, a C1-8 alkoxycarbonyl group, an aryloxycarbonyl group, or an aralkoxycarbonyl group],
or a salt thereof,
comprising the steps of:
condensing with dehydration a 3-(3-cyanophenyl)-5-formyl benzoic acid derivative represented by the following Formula (I): 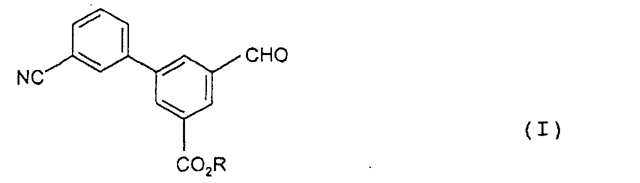
[wherein
R represents a hydrogen atom, a C1-8 alkyl group, an aryl group, or an aralkyl group]
and 4-(aminomethyl)piperidine, to obtain a 3-(3-cyanophenyl)-5-({N-[(4-piperidyl)methyl]imino}methyl)benzoic acid derivative represented by the following Formula (II): 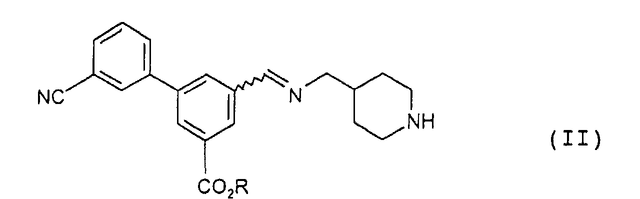 [wherein the wavy line may be, relative to the double bond, any of an E form, a Z form, or a mixture thereof at any ratio, and
R represents a hydrogen atom, a C1-8 alkyl group, an aryl group, or an aralkyl group];
protecting the secondary amine of the piperidine moiety to give a 3-(3-cyanophenyl)-5-({N-[(4-piperidyl)methyl]imino}methyl)benzoic acid derivative, having a protecting group, represented by the following Formula (III): 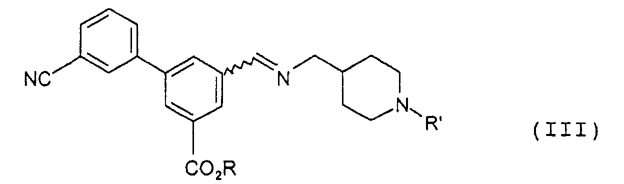 [wherein the wavy line may be, relative to the double bond, any of an E form, a Z form, or a mixture thereof at any ratio,
R represents a hydrogen atom, a C1-8 alkyl group, an aryl group, or an aralkyl group, and
R' represents a C1-8 alkylcarbonyl group, an arylcarbonyl group, a C1-8 alkoxycarbonyl group, an aryloxycarbonyl group, or an aralkoxycarbonyl group]; and then
reducing the imine moiety.

2. A process for production of a 3-(3-cyanophenyl)-5-[{[(4-piperidyl)methyl]amino}methyl)benzoic acid derivative represented by the following Formula (VIII): 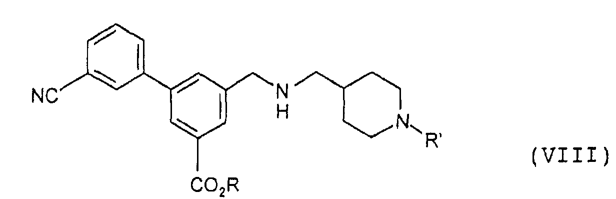 [wherein
R represents a C1-8 alkyl group, and
R' represents a C1-8 alkoxycarbonyl group]
or a salt thereof,
comprising the steps of:
condensing with dehydration a 3-(3-cyanophenyl)-5-formyl benzoic acid derivative represented by the following Formula (V): 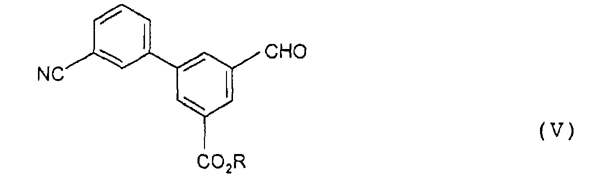
[wherein R represents a C1-8 alkyl group]
and 4-(aminomethyl)piperidine, in a toluene solution to give a 3-(3-cyanophenyl)-5-({N-[(4-piperidyl)methyl]imino}methyl)benzoic acid derivative represented by the following Formula (VI): 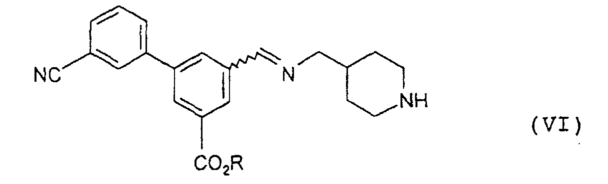 [wherein the wavy line may be, relative to the double bond, any of an E form, a Z form, or a mixture thereof at any ratio, and
R represents a C1-8 alkyl group];
protecting the secondary amine of the piperidine moiety to give a 3-(3-cyanophenyl)-5-({N-[(4-piperidyl)methyl)imino}methyl)benzoic acid derivative, having a protecting group, represented by the following Formula (VII): 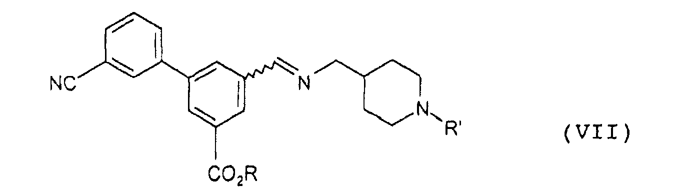 [wherein,
the wavy line may be, relative to the double bond, any of an E form, a Z form, or a mixture thereof at any ratio,
R represents a C1-8 alkyl group, and
R' represents a C1-8 alkoxycarbonyl group] and then reducing the imine moiety.

3. A process for production of a 3-(3-cyanophenyl)-5-({[(1-tert-butoxycarbonyl-4-piperidyl)methyl]amino}methyl)benzoic acid derivative represented by the following Formula (X): 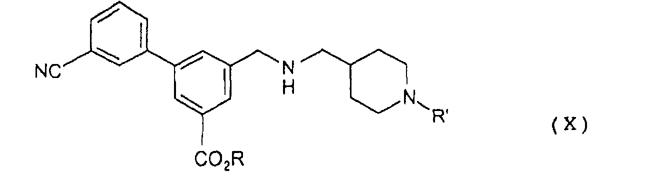 [wherein
R represents a C1-8 alkyl group, and
R' represents a tert-butoxycarbonyl group]
or a salt thereof,
comprising the steps of:
condensing with dehydration a 3-(3-cyanophenyl)-5-formyl benzoic acid derivative represented by the following Formula (V): 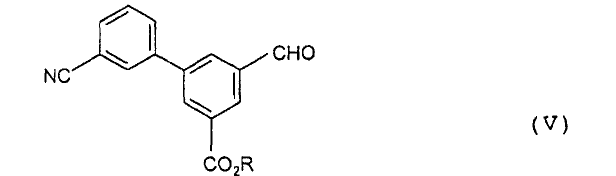
(wherein R represents a C1-8 alkyl group]
and 4-(aminomethyl)piperidine in a toluene solution to give a 3-(3-cyanophenyl)-5-({N-[(4-piperidyl)methyl]imino}methyl)benzoic acid derivative represented by the following Formula (VI): 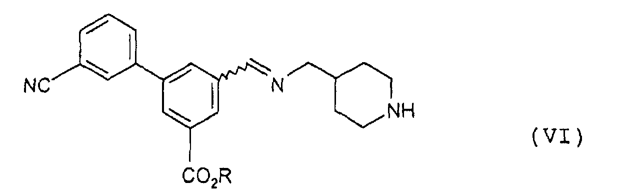 [wherein the wavy line may be, relative to the double bond, any of an E form, a Z form, or a mixture thereof at any ratio, and
R represents a C1-8 alkyl group];
protecting the secondary amine of the piperidine moiety with a tert-butoxycarbonyl group to give a 3-(3-cyanophenyl)-5-({N-[(1-tert-butoxycarbonyl-4-piperidyl)methyl]imino}methyl)benzoic acid derivative represented by the following Formula (IX): 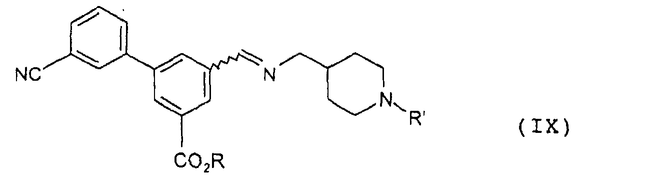 [wherein,
the wavy line may be, relative to the double bond, any of an E form, a Z form, or a mixture thereof at any ratio,
R represents a C1-8 alkyl group, and
R' represents a tert-butoxycarbonyl group]; and then
reacting the imine moiety with a borohydride complex compound.

4. A 3-(3-cyanophenyl)-5-({N-[(4-piperidyl)methyl]imino}methyl)benzoic acid derivative represented by the following Formula (II): 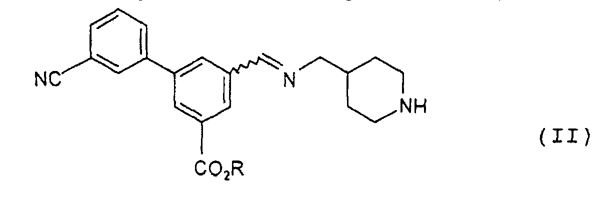 (wherein,
the wavy line may be, relative to the double bond, any of an E form, a Z form, or a mixture thereof at any ratio, and
R represents a hydrogen atom, a C1-8 alkyl group, an aryl group, or an aralkyl group],
or a salt thereof.

5. A 3-(3-cyanophenyl)-5-({N-((4-piperidyl)methyl]imino}methyl)benzoic acid derivative represented by the following Formula (III): 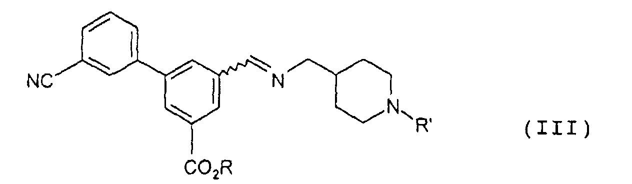 [wherein the wavy line may be, relative to the
double bond, any of an E form, a Z form, or a mixture thereof at any ratio,
R represents a hydrogen atom, a C1-8 alkyl group, an aryl group, or an aralkyl group, and
R' represents a C1-8 alkylcarbonyl group, an arylcarbonyl group, a C1-8 alkoxycarbonyl group, an aryloxycarbonyl group, or an aralkoxycarbonyl group]
or a salt thereof.

6. A 3-(3-cyanophenyl)-5-({N-[(4-piperidyl)methyl]imino}methyl)benzoic acid derivative wherein R is a C1-8 alkyl group in the Formula (II) according to claim 4, or a salt thereof.

7. A 3-(3-cyanophenyl)-5-({N-[ (1-tert-butoxycarbonyl-4-piperidyl)methyl]imino}methyl)benzoic acid derivative wherein R is a C1-8 alkyl group and R' is a tert-butoxycarbonyl group in the Formula (III) according to claim 5,
or a salt thereof.
